# EUROPEAN PATENT APPLICATION

(11) **EP 4 257 117 A1**
(43) Date of publication of application: **11.10.2023**
(21) Application number: 21901071.7
(22) Date of filing: 03.12.2021
(51) Int. Cl.: A61K 9/00, A61K 47/24, A61K 47/10, A61K 31/565, A61P 35/00

(54) **SUSTAINED-RELEASE PHARMACEUTICAL COMPOSITION OF FULVESTRANT AND METHOD FOR PREPARING SAME**

(30) Priority: 04.12.2020 KR 20200168867
(71) Applicant: Samyang Holdings Corporation, Seoul 03129 (KR)
(72) Inventor: LEE, Jae Young, Yongin-si Gyeonggi-do 16838 (KR); KIM, Dong Sik, Suwon-si Gyeonggi-do 16349 (KR); YUN, Min Hyuk, Yongin-si Gyeonggi-do 16899 (KR)
(74) Representative: Wohlfahrt, Jan Günther
(86) International application number: PCT/KR2021/018233
(87) International publication number: WO 2022/119383

(57) **Abstract**

The present invention relates to a sustained-release pharmaceutical composition comprising fulvestrant and a method for preparing same and, more specifically, to a pharmaceutical composition and a method for preparing same, in which, by comprising, in the pharmaceutical composition, a combination of lecithin, a C2-C5 alcohol, and polyethylene glycol, along with fulvestrant, which is a poorly soluble drug, the solubility of the drug is improved, thus increasing the concentration of the drug in a formulation, and thus, pharmacological therapeutic effects equivalent to those of conventional fulvestrant formulations are exhibited and the number of administrations can be reduced, thus improving the inconvenience of conventional formulations and increasing the convenience of patients and providers.

## Description

### TECHNICAL FIELD

The present invention relates to a pharmaceutical composition comprising Fulvestrant for sustained release and a method for preparing the same, and more specifically, it relates to a pharmaceutical composition and a method for preparing the same wherein the pharmaceutical composition comprises lecithin, C2 to C5 alcohol and polyethylene glycol in combination together with Fulvestrant, which is a poorly soluble drug, and thus the solubility of the drug can be improved and the concentration of the drug in the formulation can be increased, and thereby, in comparison with the existing Fulvestrant formulations, the pharmaceutical composition shows the equivalent pharmacological effect of treatment with reduced number of administration, and thus the inconvenience of the existing formulations can be improved and the convenience for patients and providers can be increased.

### BACKGROUND ART

The chemical name of Fulvestrant is 7-alpha-[9-(4,4,5,5,5-penta fluoropentylsulphinyl) nonyl]estra-1,3,5-(10)-triene-3,17beta- diol, which has the following chemical structure:

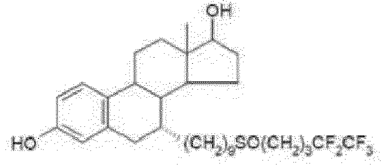

Fulvestrant is a drug used to treat hormone receptor-positive (ER+), HER2-negative (HER2-) advanced breast cancer, and is the only SERD (selective estrogen receptor degrader) drug whose approval has been completed. Fulvestrant is marketed under the product name Faslodex^{®} Injection (AstraZeneca) which is a formulation containing 250 mg of Fulvestrant in a 5 mL injection solution, and in principal, a dose of 500 mg (2 prefilled syringes) is administered intramuscularly once every 2 weeks for the first one month, and thereafter the same amount is administered once a month.

When administering Faslodex^{®} Injection to a patient, the administration method is set to inject into both gluteal (buttock) muscles for 1 to 2 minutes each. However, this method causes considerable pain to the patient receiving the administration and considerable inconvenience to the provider who proceeds with the administration. It is interpreted that the administration method is set so because the total volume of the injection solution is 10 mL, which far exceeds the maximum volume (about 5 mL) that can be administered at one time when administered intramuscularly.

Fulvestrant has extremely low solubility in aqueous solution and, according to FDA data, it is known as "practically insoluble" in pure water (According to the prescription of Faslodex^{®} Injection, the saturation solubility of Fulvestrant was found to be about 90 mg/mL at room temperature and about 65 mg/mL in refrigerated storage, and the concentration of the product is 50 mg/mL as Fulvestrant). Therefore, when formulating Fulvestrant, a solubilizing agent should be used necessarily, and in case of Faslodex^{®} Injection, ethanol and benzyl alcohol have been used as major solubilizing agents, and they all correspond to organic solvents.

Faslodex^{®} Injection is an oil depot formulation, and among the additives, castor oil component accounts for the largest part. Oil depot is a formulation which can be found commonly in sustained-release injections, and most of matrix thereof consists of oil, and it is characterized by maintaining a liquid phase in the body, differently from sustained-release formulations using polymers such as in situ forming gel, in situ polymer precipitation, polymeric implant formulations, etc. Oil Depot is a formulation that can be injected both subcutaneously and intramuscularly, and Faslodex^{®} Injection is used only for intramuscular injection.

When formulating Fulvestrant, the type and amount of oil and the ratio of organic solvent, etc. are very important and accordingly the solubility of Fulvestrant in the matrix is determined, and in addition they become major factors in determining the characteristics of sustained release (release rate, initial release amount, etc.).

### CONTENTS OF THE INVENTION

### PROBLEMS TO BE SOLVED

The present invention is to resolve the problem of excessively large administration volume of the existing Fulvestrant formulations, and the purpose of the present invention is to provide a composition comprising Fulvestrant capable of improving the solubility of Fulvestrant in the formulation and thus increasing the drug concentration, and a method for preparing the same.

### TECHNICAL MEANS

One aspect of the present invention provides a sustained-release pharmaceutical composition for treating cancer, comprising: Fulvestrant as an active ingredient; lecithin; C2 to C5 alcohol; and polyethylene glycol.

The other aspect of the present invention provides a method for preparing a sustained-release pharmaceutical composition for treating cancer, the method comprising: (1) a step comprising dissolving Fulvestrant and lecithin in C2 to C5 alcohol; and (2) a step of adding polyethylene glycol to the product of the step (1).

### EFFECT OF THE INVENTION

The sustained-release pharmaceutical composition of Fulvestrant provided according to the present invention shows physicochemical equivalence to the existing Fulvestrant formulations and secures equivalence in non-clinical studies and bioequivalence tests, showing equivalent pharmacological therapeutic effects, and in comparison with the existing formulations, it can increase the concentration of Fulvestrant so that the number of administrations can be reduced (the existing 2 administrations can be reduced to 1 administration), and thereby the inconvenience of the existing formulations can be improved and the convenience for patients and providers can be increased.

### PREFERRED MODE FOR CARRYING OUT THE INVENTION

In the present entire specification, unless mentioned especially otherwise, "comprising" or "containing" refers to comprising a certain constitutional element (or constitutional component) without special limitation, and it is not interpreted as excluding addition of other constitutional element (or constitutional component).

The present invention is explained in more detail below.

One aspect of the present invention relates to a sustained-release pharmaceutical composition for treating cancer, comprising: Fulvestrant as an active ingredient; lecithin; C2 to C5 alcohol; and polyethylene glycol.

In an embodiment, the amount of Fulvestrant comprised in the pharmaceutical composition may be, based on total of 100 % by weight of the composition, for example, 5 % by weight or more, 6 % by weight or more, 7 % by weight or more, 8 % by weight or more, or 9 % by weight or more, and it also may be 20 % by weight or less, 18 % by weight or less, 15 % by weight or less, 13 % by weight or less, or 11 % by weight or less, but it is not limited thereto.

In an embodiment, the amount of the lecithin comprised in the pharmaceutical composition may be, based on 1 part by weight of the C2 to C5 alcohol, for example, 0.05 part by weight or more, 0.1 part by weight or more, or 0.2 part by weight or more, and it also may be 1 part by weight or less, 0.8 part by weight or less, or 0.5 part by weight or less, but it is not limited thereto. If the amount of the lecithin is less than the above range, the solubility after administration may be reduced, and if it is greater than the above range, the viscosity increases so that the administration as an injection formulation may not be appropriate.

In the present specification, "C2 to C5 alcohol" means alcohol with 2 to 5 total carbon atoms in the molecule.

In an embodiment, the C2 to C5 alcohol may be ethanol.

In an embodiment, the amount of the C2 to C5 alcohol comprised in the pharmaceutical composition may be, based on total of 100 % by weight of the composition, for example, 5 % by weight or more, 7 % by weight or more, or 10 % by weight or more, and it also may be 30 % by weight or less, 25 % by weight or less, or 20 % by weight or less, but it is not limited thereto.

In an embodiment, the amount of the polyethylene glycol comprised in the pharmaceutical composition may be, based on 1 part by weight of the C2 to C5 alcohol, for example, 0.1 part by weight or more, 0.2 part by weight or more, or 0.5 part by weight or more, and it also may be 1.5 parts by weight or less, 1.2 parts by weight or less, or 1.0 part by weight or less, but it is not limited thereto. If the amount of the polyethylene glycol is less than the above range, the formulation may become unstable and the amount of active ingredient precipitated may increase, and if it is greater than the above range, after the administration, the initial release of the active ingredient may increase, resulting in poor sustained release.

The polyethylene glycol may have a weight average molecular weight of, for example, 200,000 to 1,000,000 g/mol, but it is not limited thereto.

In an embodiment, the pharmaceutical composition may further comprise vegetable oil.

In an embodiment, the vegetable oil may be castor oil.

In an embodiment, when the pharmaceutical composition further comprises vegetable oil, the amount thereof may be, based on 1 part by weight of the C2 to C5 alcohol, for example, 1 to 10 parts by weight, and more concretely 1 to 5 parts by weight, and still more concretely 2 to 4 parts by weight, but it is not limited thereto. Since the vegetable oil serves as a matrix in the formulation of the present invention, if its amount is less than the above range, the sustained release property is reduced, and if the amount is greater than the above range, the formulation may become unstable or the release rate may decrease significantly.

In an embodiment, the pharmaceutical composition may further comprise benzyl alcohol, benzyl benzoate, or a combination thereof.

The benzyl alcohol and/or benzyl benzoate can prevent a significant increase in the Cmax value of the composition and thus can be helpful in reducing unexpected side effects of the drug. However, if the amount of the benzyl alcohol and benzyl benzoate is too large, the the solubility may be lowered and thereby the stability of the sustained-release formulation may decrease.

In an embodiment, when the pharmaceutical composition further comprises benzyl alcohol, the amount thereof may be, based on 1 part by weight of the C2 to C5 alcohol, for example, 0.1 part by weight or more, 0.2 part by weight or more, or 0.5 part by weight or more, and it also may be 2 parts by weight or less, 1.5 parts by weight or less, or 1.0 part by weight or less, but it is not limited thereto.

In an embodiment, when the pharmaceutical composition further comprises benzyl benzoate, the amount thereof may be, based on 1 part by weight of the C2 to C5 alcohol, for example, 0.1 part by weight or more, 0.2 part by weight or more, or 0.5 part by weight or more, and it also may be 2 parts by weight or less, 1.5 parts by weight or less, or 1.0 part by weight or less, but it is not limited thereto.

In an embodiment, when the pharmaceutical composition further comprises a combination of benzyl alcohol and benzyl benzoate, the weight ratio of benzyl alcohol to benzyl benzoate in the composition may be, for example, 0.1 to 10 : 1, or 0.2 to 5 : 1, or 0.5 to 3 : 1, or 0.5 to 1.5 : 1, but it is not limited thereto.

In an embodiment, the pharmaceutical composition is an injection formulation, and more concretely an intramuscular injection formulation.

In an embodiment, the injection force of the injection formulation may be 0.5 to 10 kgf.

In an embodiment, the concentration of Fulvestrant in the injection formulation may be 90 to 300 mg/mL.

In an embodiment, the *in vitro* release time using a dialysis bag of the injection formulation may be 14 to 60 days.

In an embodiment, the pharmaceutical composition is for treating cancer, more concretely for treating breast cancer, and still more concretely for treating advanced breast cancer.

The other aspect of the present invention relates to a method for preparing a sustained-release pharmaceutical composition for treating cancer, the method comprising: (1) a step comprising dissolving Fulvestrant and lecithin in C2 to C5 alcohol; and (2) a step of adding polyethylene glycol to the product of the step (1).

In an embodiment, the C2 to C5 alcohol may be ethanol.

In an embodiment, the step (1) may further comprise partially removing C2 to C5 alcohol from the mixture of Fulvestrant, lecithin and C2 to C5 alcohols.

The removal of C2 to C5 alcohol can be conducted, for example, at 30 to 80 °C by using a rotary evaporator, but it is not limited thereto.

In an embodiment, the product of the step (1) may be a lecithin film containing Fulvestrant.

In an embodiment, when the step (1) further comprises partially removing C2 to C5 alcohol from the mixture of Fulvestrant, lecithin and C2 to C5 alcohols, the method for preparing the pharmaceutical composition may further comprise, after the step (2), a step of further adding C2 to C5 alcohol to the product of the step (2).

In an embodiment, the method for preparing the pharmaceutical composition may further comprise one or more steps of simultaneously or sequentially adding C2 to C5 alcohol and vegetable oil to the product of the step (2).

Preferable examples are provided below in order to facilitate understanding of the present invention. However, the following examples are only to illustrate the present invention, and the scope of the present invention is not limited thereby in any manner.

### [EXAMPLES]

### Examples 1 to 3: Preparation of Fulvestrant formulation

Fulvestrant, lecithin and ethanol were placed in a round bottom flask and mixed. The solvent was evaporated by using a rotary evaporator at 50°C to obtain a lecithin film. The amount of ethanol remaining in the obtained lecithin film was weighed. Polyethylene glycol (PEG) was added to the round bottom flask, and it was kept in rotation until the lecithin film was completely dissolved. Ethanol including the amount remaining in the mixture, and a certain amount of benzyl alcohol (BA), benzyl benzoate (BB) or a combination thereof were added and mixed. Castor oil was then added to the mixture to adjust the weight of the final solution to 100% of the total of the components except Fulvestrant. The amount of each component added is shown in Table 1 below. As Control group, a formulation of the components of Faslodex^{®} (containing Fulvestrant as an active ingredient) except for Fulvestrant was used.

**[Table 1] (Unit of amount: % by weight)**

| Example | BA (%) | BB (%) | Lecithin (%) | PEG (%) | Ethanol (%) | Castor oil (%) |
|---|---|---|---|---|---|---|
| Control | 10 | 15 | - | - | 10 | 65 |
| 1 | 12.5 | 12.5 | 5 | 10 | 15 | 45 |
| 2 | 18.75 | 6.25 | 5 | 10 | 15 | 45 |
| 3 | 6.25 | 18.75 | 5 | 10 | 15 | 45 |

### Test Example: Measurement of saturated solubility of Fulvestrant according to matrix composition

For the formulations of Examples 1 to 3 and Control group, saturated solubility of Fulvestrant therein was measured at room temperature (25°C). The saturation solubility was measured for three (3) samples for each Example and Control group, the average value was obtained. The results are shown in Table 2 below.

**[Table 2]**

| Example | Saturated solubility (mg/mL) |
|---|---|
| Control | 95 |
| 1 | 126.3 |
| 2 | 116.1 |
| 3 | 110.3 |

## Claims

1. A sustained-release pharmaceutical composition for treating cancer, comprising: Fulvestrant as an active ingredient; lecithin; C2 to C5 alcohol; and polyethylene glycol.

2. The pharmaceutical composition of claim 1, further comprising vegetable oil.

3. The pharmaceutical composition of claim 2, wherein the vegetable oil is castor oil.

4. The pharmaceutical composition of claim 1, wherein the C2 to C5 alcohol is ethanol.

5. The pharmaceutical composition of claim 2, wherein the amount of vegetable oil is 1 to 10 parts by weight, based on 1 part by weight of the C2 to C5 alcohol.

6. The pharmaceutical composition of claim 1, further comprising benzyl alcohol, benzyl benzoate, or a combination thereof.

7. The pharmaceutical composition of any one of claims 1 to 6, wherein the composition is an intramuscular injection formulation.

8. A method for preparing a sustained-release pharmaceutical composition for treating cancer, the method comprising:
(1) a step comprising dissolving Fulvestrant and lecithin in C2 to C5 alcohol; and
(2) a step of adding polyethylene glycol to the product of the step (1).

9. The method for preparing a pharmaceutical composition of claim 8, wherein the product of the step (1) is a lecithin film containing Fulvestrant.

10. The method for preparing a pharmaceutical composition of claim 8 or 9, further comprising one or more steps of simultaneously or sequentially adding C2 to C5 alcohol and vegetable oil to the product of the step (2).
